# EUROPÄISCHE PATENTSCHRIFT

(11) **EP 2 681 264 B1**
(45) Veröffentlichungstag und Bekanntmachung des Hinweises auf die Patenterteilung: **02.08.2017**
(21) Anmeldenummer: 12705868.3
(22) Anmeldetag: 27.02.2012
(51) Int. Cl.: C08J 3/12, A61K 9/28

(54) **HERSTELLUNG VON PULVERFÖRMIGEN ÜBERZUGSMITTELN FÜR STABILE PROTEKTIVE ÜBERZÜGE FÜR PHARMAZEUTISCHE DOSIERUNGSFORMEN**
PROCESS FOR MAKING POWDER COATING COMPOSITIONS FOR STABLE PROTECTING COATING FOR PHARMACEUTICAL DOSAGE FORMS
PROCÉDÉ DE PRÉPARATION DE COMPOSITIONS DE REVÊTEMENTS EN POUDRE POUR DES REVÊTEMENTS PROTECTEURS STABLES DE FORMES DE DOSAGE PHARMACEUTIQUE.

(30) Priorität: 28.02.2011 EP 11156209
(43) Veröffentlichungstag der Anmeldung: 08.01.2014
(73) Patentinhaber: BASF SE, 67056 Ludwigshafen am Rhein (DE)
(72) Erfinder: KOLTER, Karl, 67117 Limburgerhof (DE); ANGEL, Maximilian, 67105 Schifferstadt (DE); LINNER, Bernhard, 67240 Bobenheim-Roxheim (DE)
(74) Vertreter: BASF IP Association
(86) Internationale Anmeldenummer: PCT/EP2012/053232
(87) Internationale Veröffentlichungsnummer: WO 2012/116941

(56) Entgegenhaltungen:
- DE-A1- 3 049 179
- US-A- 4 816 558
- US-A1- 2004 152 867
- US-A1- 2011 033 532
- DATABASE WPI Week 200220 Thomson Scientific, London, GB; AN 2002-154448 XP002673622, & WO 01/88021 A1 (MITSUBISHI RAYON CO LTD) 22. November 2001 (2001-11-22)
- DATABASE COMPENDEX [Online] ENGINEERING INFORMATION, INC., NEW YORK, NY, US; Juli 2007 (2007-07), XIE D-L ET AL: "Influences of inlet and outlet drying temperatures on re-dispersible EVA polymer powders", XP002673623, Database accession no. E20073810818540 & HUANAN LIGONG DAXUE XUEBAO/JOURNAL OF SOUTH CHINA UNIVERSITY OF TECHNOLOGY (NATURAL SCIENCE) JULY 2007 SOUTH CHINA UNIVERSITY OF TECHNOLOGY CN, Bd. 35, Nr. 7, Juli 2007 (2007-07), Seiten 88-92,

## Beschreibung

Die vorliegende Erfindung betrifft die Herstellung von pulverförmigen Überzugsmittels für stabile protektive Überzüge für pharmazeutische Dosierungsformen, die zwecks Geschmacksmaskierung oder zum Schutz gegen Feuchtigkeit mit einem Filmüberzug auf Basis eines kationischen Polymers, welches mittels radikalischer Emulsionspolymerisation eines N,N-Diethylaminoethylmethacrylat enthaltenden Monomergemischs erhalten wird, versehen werden, durch Überführen einer wässrigen Dispersion mittels Sprühverfahren in ein freifliessendes Pulver mit guter Redispergierbarkeit. Weiterhin betrifft die Erfindung Verfahren zur Redispergierung der Pulver.
Die DE-AS 2512238 lehrt zur Bereitstellung von Bindemitteln für Arzneimittelüberzüge mit geringem Restmonomerengehalt die Verwendung eines durch Sprühtrocknen einer Polymerdispersion erhaltenen Pulvers zur Herstellung von Überzugslösungen für diese Arzneiformen. Bezüglich der zur Sprühtrocknung eingesetzten Dispersionen wird auf die DE 1090381, DE 1219175 und DE 2135073 Bezug genommen.

Die DE 3049179 A1 ist eine Zusatzanmeldung zur DE 2512238 und betrifft die Verwendung eines durch Sprühtrocknen nach der Lehre des letztgenannten Dokuments gewonnenen Pulvers in Form einer wässrigen Suspension, die zusätzlich ein Weichmachungsmittel enthält, zur Herstellung von Überzügen durch Thermogelierung.

Die WO 00/05307 beschäftigt sich mit der Bereitstellung von Überzugs- und Bindemitteln für Arzneiformen, die (Meth)acrylat-Copolymere enthalten, die Monomerreste mit tertiären Aminogruppen aufweisen, wobei eine einfache trockene oder wässrige Weiterverarbeitung möglich sein soll. Dazu lehrt dieses Dokument ein Verfahren, bei dem man (a) ein Copolymer aus C₁-C₄-Estern der (Meth)acrylsäure und (Meth)acrylat-Monomeren, die tertiäre Ammoniumgruppen aufweisen, (b) einen Weichmacher und (c) einen Emulgator mit einem HLB-Wert von mindestens 14 miteinander vermengt und daraus das Überzugs- oder Bindemittel durch Schmelzen, Gießen, Ausstreichen oder Aufsprühen herstellt, wobei das Copolymer (a) in Pulverform mit einer mittleren Teilchengröße von 1 bis 40 µm eingebracht wird. Die dabei erzielte Verarbeitbarkeit wird der Bereitstellung des Copolymeren (a) in Pulverform mit extrem geringer Korngröße zugeschrieben.

Die WO 02/067906 betrifft Überzugs- und Bindemittel mit verbesserter Wasserdampfdurchlässigkeit gegenüber den in der WO 00/05307 beschriebenen. Dabei erfolgt die Herstellung der Überzugs- und Bindemittel mit einer Mischung, die (a) ein Copolymer aus C₁-C₄-Estern der (Meth)acrylsäure und weitere (Meth)acrylat-Monomere mit funktionellen tertiären Ammoniumgruppen in Pulverform mit einer mittleren Teilchengröße von 1 bis 40 µm, (b) einen Emulgator mit einem HLB-Wert von mindestens 14 und (c) eine C₁₂-C₁₈-Monocarbonsäure oder eine C₁₂-C₁₈-Hydroxylverbindung enthält.

Die WO 2004/019918 beschreibt Überzugs- und Bindemittel, die bezüglich ihrer Zusammensetzung den in der WO 00/05307 und WO 02/067906 beschriebenen entsprechen.

Gemäß der US 6,696,085 B2 soll ein Methacrylsäure-Copolymer Typ C als Zerfallsmittel eingesetzt werden. Das Methacrylsäure-Copolymer Typ C ist ein magensaft-resistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

In der EP88951 A2 ist ein Verfahren zum Überziehen von Arzneimitteln mittels eines in Wasser dispergierten Überzugsmittels auf Basis von Emulsionspolymerisaten beschrieben, wo bei die Überzugsmittel partiell in Salzform vorliegen können. Die Überzugsmittel können auch aus redispergierten Pulvern erhalten werden, wobei als grundsätzlich geeignete Methoden die Verfahren der Sprühtrocknung und der Gefriertrocknung genannt werden. Allerdings wird in diesem Zusammenhang auch ausgeführt, dass die Gefriertrocknung auch an der unteren Grenze des Bereichs geeigneter Glastemperaturen noch anwandbar sei. Konkret beschreiben werden entweder durch Gefriertrocknung erhaltene Pulver oder ein sprühgetrocknetes Produkt aus 30 % Methacrylsäure und 70 % Methylmethacrylat, dass aufgrund seiner Zusammensetzung eine hohe Glasübergangstemperatur aufweist.

In der WO 97/42255 ist die Sprühtrocknung von in wässriger Lösung redispergierbaren Polymerpulvern, enthaltend freie säuren- oder basen-gruppentragende Copolymere, durch Sprühtrocknung beschrieben, wobei vor der Sprühtrocknung die pH-Werte der Dispersionen mit Hilfe eine Puffersystems eingestellt werden müssen.

In der EP 262326 A2 ist ein Verfahren zur Herstellung eines redispergierbaren Kunststoffpulvers beschrieben, bei dem eine wässrige Dispersion eines Copolymers aus (Meth)acrylsäure und (Meth)acrylsäureestern mit einer Mindestfilmbildetemperatur unter 60°C und einer dynamischen Einfriertemperatur unter 150°C so sprühgetrocknet wrid, dass die Eingangstemperatur des Trocknungsgases über der Mindestfilmbildetemperatur und unter der Einfriertemperatur liegt.

Aus der WO 2009/016258 ist die Herstellung der wässrigen Polymerdispersionen kationischer Polymere auf Basis von N,N-Diethylaminoethylmethacrylat wie sie erfindungsgemäß zum Einsatz kommen und deren Verwendung zum Überziehen von Arzneistoffen bekannt. Der Einsatz in Pulverform ist nur ganz allgemein erwähnt. Hinzu kommt, dass die Polymere aufgrund ihrer niedrigen Glasübergangstemperatur eine unerwünschte Neigung zur Agglomeration zeigen können und damit verarbeitungstechnisch hohe Anforderungen stellen.

Der vorliegenden Erfindung liegt die Aufgabe zu Grunde, freifliessende pulverförmige Filmüberzugsmittel mit guter Redispergierbarkeit in Wasser zu finden, die für pharmazeutische Dosierungsformen, die auch bei längerer oder temperaturbelastender Lagerung keine Veränderung im Freisetzungsverhalten aufweisen, geeignet sind. Eine besondere Anforderung an die zu findenden redispergierten Überzugsmittel ist die Erzeugung von kleinteiligen Dispersionen mit engen Teilchengrößenverteilungen und die Vermeidung von Koagulatbildung.

Demgemäß wurde ein Verfahren zur Herstellung von pulverförmigen Überzugsmitteln aus wässrigen Polymerdispersionen, enthaltend
i) als Komponente A ein durch radikalische Polymerisation erhaltenes Polymer aus
   a) N,N-Diethylaminoethylmethacrylat, und
   b) wenigstens eine radikalisch polymerisierbare Verbindung, ausgewählt unter Estern α,β-ethylenisch ungesättigter Mono- und Dicarbonsäuren mit C₁-C₈-Alkanolen, gefunden, welches dadurch gekennzeichnet ist, dass die wässrige Polymerdispersion durch Sprühverfahren in Gegenwart eines Trocknungsgases in freifliessende Pulver überführt wird, wobei die Eingangstemperatur des Trocknungsgases in die Sprühvorrichtung mindestens 20°C über der Glasübergangstemperatur und mindestens 20°C über der Mindestfilmbildetemperatur des Polymers liegt und die Ausgangstemperatur des Trocknungsgases aus der Sprühvorrichtung bei 40 bis 85°C gehalten wird.

Gemäß einer weiteren Ausführungsform liegt die Eingangstemperatur des Trockengases mindestens 20°C über der dynamischen Einfriertemperatur und mindestens 20°C über der dynamischen Einfriertemperatur und mindestens 20°C über der Mindestfilmbildetemperatur des Polymers und wobei die Ausgangstemperatur des Trocknungsgases aus der Sprühvorrichtung bei 40 bis 85°C beträgt.

Vorzugsweise liegt die Eingangstemperatur des Trocknungsgases in die Sprühvorrichtung mindestens 40°C über der Glasübergangstemperatur und mindestens 40°C über der Mindestfilmbildetemperatur des Polymers.

Gemäß einer Ausführungsform der Erfindung liegt die Eingangstemperatur des Trockengases mindestens 20°C über der dynamischen Einfriertemperatur und mindestens 40°C über der dynamischen Einfriertemperatur und mindestens 40°C über der Mindestfilmbildetemperatur des Polymers. Gemäß einer weiteren Ausführungsform und/oder mindestens 40°C über der dynamischen Einfriertemperatur.

Für alle oben genannten Ausführungsformen beträgt die Ausgangstemperatur des Trocknungsgases vorzugsweise 45 bis 70°C

Gemäß einer weiteren bevorzugten Ausführungsform erfolgt die Überführung in freifliessende Pulver durch agglomerierende Sprühtrocknung.
Gemäß einer weiteren bevorzugten Ausführungsform werden die Polymere vor oder nach dem Sprühverfahren mit Säuren teilneutralisiert.

Eine weitere Ausführungsform betrifft die Durchführung eines Sprühverfahrens in Gegenwart von weiteren Polymeren und/oder weiteren Hilfsstoffen.

Weiterhin wurde die Verwendung der so erhaltenen Pulver als pharmazeutische Überzugsmittel gefunden. Vorzugsweise werden die Überzugsmittel durch Redispergierung in Wasser erhalten, wobei das durch ein Sprühverfahren erhaltene Pulver unter Einsatz von niedrig-scherenden Rührvorrichtungen bei Umdrehungen bis 1000 Upm redispergiert wird. Überraschenderweise können auch hoch-scherende Dispergiervorrichtungen bei Umdrehungen von > 5000 Upm verwendet werden. Dies kann erfindungsgemäß erfolgen, ohne dass die bei der Redisperdierung gebildeten feinen Teilchen agglomerieren und die Zubereitung koaguliert.
Freifliessende Pulver im Sinne der vorliegenden Erfindung bedeutet, dass die Pulver bei der Bestimmung der Fließfähigkeit nach der DIN ISO 4324 unter Verwendung des Gerätes nach Pfrengle ohne Rührhilfe frei und vollständig aus dem Trichter ausfließen.

Die für die Sprühverfahren verwendeten Überzugsmittel basieren auf wässrigen Polymerdispersionen, welche durch radikalische Emulsionspolymerisation eines Monomergemischs M), enthaltend
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
in einem wässrigen Medium bei einem pH-Wert von wenigstens 8, erhalten werden.

Die Überzugsmittel in Form von wässrigen Polymerdispersionen enthalten vorzugsweise keine zusätzlichen organischen Lösungsmittel.

Erfindungsgemäß dienen die Überzugsmittel zur Herstellung von pharmazeutischen Dosierungsformen, die im sauren Milieu des Magens schnell freisetzend sein sollen. D.h. die erfindungsgemäßen Überzüge sind magensaftlöslich. Schnell freisetzend bedeutet in diesem Zusammenhang, dass nach 60 min mindestens 80 % des Wirkstoffs freigesetzt sind. Erfindungsgemäß erhaltene Überzüge sollen sich in Mundhöhle und Rachen im neutralen oder nahezu neutralen Milieu des Speichels nicht auflösen.
Die erfindungsgemäßen Überzugsmittel können zur Geschmacksmaskierung oder zum Schutz vor Feuchtigkeit verwendet werden. Die Wasserdampfpermeabilität der Überzüge ist sehr niedrig, wodurch feuchtigkeitsempfindliche Wirkstoffe geschützt werden.

Zur Herstellung der Polymerisate durch radikalische Emuslionspolymerisation wird hiermit ausdrücklich auf die Offenbarung der WO 2009/016258 Bezug genommen, in der die Herstellung und bevorzugte Ausführungsformen bezüglich Herstellung sowie Zusammensetzung ausführlich beschrieben sind.

In einer besonders bevorzugten Ausführungsform des erfindungsgemäßen Verfahrens wird eine Polymerdispersion eingesetzt, die aus einem Monomergemisch M), das aus
- 43 bis 47 Gew.- %, bezogen auf das Gesamtgewicht der zur Polymerisation eingesetzten Monomere, N,N-Diethylaminoethylmethacrylat a), und
- 53 bis 57 Gew.- %, bezogen auf das Gesamtgewicht der zur Polymerisation eingesetzten Monomere, wenigstens einer Verbindung b), insbesondere Methylmethacrylat,
besteht, erhalten wird.

Die in den Dispersionen enthaltenen Polymere weisen vorzugsweise ein mittels Gelpermeationschromatographie bestimmtes mittleres Molekulargewicht M_{w} im Bereich von 30000 bis 500000, besonders bevorzugt 60000 bis 140000, insbesondere 80000 bis 120000 g/mol, auf.

Die in den Dispersionen Pd) enthaltenen Polymere weisen vorzugsweise einen K-Wert (bestimmt nach Fikentscher an einer 1%igen Lösung in N-Methylpyrrolidon (NMP)) im Bereich von 40 bis 60 auf.

Die Glasübergangstemperatur T_{G} bestimmt mittels DSC "Differential Scanning Calorimetry" liegt vorzugsweise in einem Bereich von 40 bis 70°C, besonders bevorzugt 52 bis 62°C. Dabei werden die Proben zunächst auf 150°C aufgeheizt und dann von 150°C schnell abgekühlt. Die Messung der Glasübergangstemperatur erfolgt mit einer Heizrate von 20°K/min.
Die Mindestfilmbildetemperatur wird nach der in der DIN ISO 2115 beschriebenen Methode bestimmt und liegt im Bereich von 40 bis 70°C, vorzugsweise 50 bis 65°C. Die Messgenauigkeit der Methode liegt im Bereicht von +/- 5°C.

Bei den in den Dispersionen enthaltenen Polymeren handelt es sich im Wesentlichen um statistische Copolymere.

Der mittlere Teilchendurchmesser der in der Polymerdispersion enthaltenen Polymerteilchen (bestimmt mittels analytischer Ultrazentrifuge) liegt vorzugsweise in einem Bereich von 70 bis 200 nm, besonders bevorzugt von 80 bis 150 nm, insbesondere von 90 bis 120 nm Die Teilchengrößenverteilung ist vorzugsweise im Wesentlichen unimodal.

Der LD-Wert der erfindungsgemäßen Dispersionen, bestimmt an einer 0,01 %igen Dispersion in Wasser (2,5 cm Küvette, weißes Licht) beträgt vorzugsweise wenigstens 70 %, besonders bevorzugt wenigstens 80 %. Die Bestimmung der Lichtdurchlässigkeit wird z. B. in Dieter Distler, Wässrige Polymerdispersionen, Wiley-VCH (1999), S. 40, beschrieben.

Der Feststoffgehalt der erfindungsgemäß für die Sprühverfahren eingesetzten Dispersionen beträgt vorzugsweise 10 bis 50 Gew.-%, besonders bevorzugt 20 bis 40 Gew.-%. Im Falle einer vorherigen Reinigung der Dispersion mittels Ultrafiltration weisen die erfindungsgemäß eingesetzten Dispersionen vorzugsweise vor und nach der Ultrafiltration Feststoffgehalte auf, die in diesen Bereichen liegen. Selbstverständlich ist es ebenfalls möglich, eine verdünnte Polymerdispersion vor dem Sprühverfahren einer Aufkonzentrierung durch Ultrafiltration zu unterziehen.

Die erfindungsgemäß zur Geschmacksmaskierung verwendeten redispergierbaren Polymerpulver weisen in Wasser bei einem Feststoffgehalt von 20 Gew.-% niedrige Viskositäten von vorzugsweise kleiner 300 mPas, besonders bevorzugt kleiner 200 mPas und insbesondere kleiner 100 mPas auf (Werte bestimmt mittels Brookfield-Viskosimeter bei 20 °C und 100 s⁻¹). Solche Viskositäten sind für viele Anwendungen von besonderer Bedeutung.

Erfindungsgemäß erfolgt die Überführung der wässrigen Polymerdisperionen in die Pulverform mittels Sprühverfahren. Als Sprühverfahren eignen sich prinzipiell die Sprühtrocknung, die agglomerierende Sprühtrocknung, die Sprühgranulation (Sprühwirbelschichttrocknung) oder die Sprühagglomeration.

Die im Folgenden genannten Bedingungen für die Durchführung der Zerstäubung und Trocknung beziehen sich auf alle prinzipiell durchführbaren Ausführungsformen des Sprühverfahrens, sei es normale Sprühtrocknung, Sprühgranulation oder agglomerierende Sprühtrocknung.

Die Zerstäubung erfolgt vorzugsweise als hydrodynamische Zerstäubung durch Flüssigkeitsdruck oder Luftdruck über Düsen wie beispielsweise Einstoff- oder Mehrstoff-Düsen oder über Zerstäubungsscheiben.

Als Sprühvorrichtungen eignen sich herkömmliche Sprühtürme, in die die zu zerstäubende Polymerdispersion von oben eingeführt wird. Die erhaltenen Polymerpulver können am unteren Ende ausgetragen werden und in einem nachgeschalteten Zyklon vom Trocknungsgasstrom abgeschieden werden.

Als Trocknungsgase können Luft oder inerte Gase wie Stickstoff, Argon oder Helium verwendet werden. Die Trocknungsgase können im Gegenstrom oder im Gleichstrom zu den durch die Zerstäubung erzeugten Flüssigkeitströpfchen durch die Sprühvorrichtung geleitet werden. Vorzugsweise wird das Trocknungsgas im Gleichstrom eingesetzt. Die Eingangstemperatur des Trocknungsgases wird mindestens 20°C, vorzugsweise mindestens 40°C über der Glasübergangstemperatur und gemäß einer Ausführungsform auch mindestens 20°C bevorzugt mindestens 40°C, über der dynamischen Einfriertemperatur und mindestens 20°C, bevorzugt mindestens 40°C, über der Mindestfilmbildetemperatur des Polymers gehalten. Die Eingangstemperatur des Trocknungsgases in die Sprühvorrichtung wird besonders bevorzugt bei 100 bis 140°C und die Ausgangstemperatur des Trocknungsgases aus der Sprühvorrichtung bei 45 bis 70°C gehalten. Ganz besonders bevorzugt wird die Eingangstemperatur des Trocknungsgases in die Sprühvorrichtung bei 110 bis 130°C und die Ausgangstemperatur des Trocknungsgases aus der Sprühvorrichtung bei 50 bis 60°C gehalten. Die Ausgangstemperatur des Trocknungsgases liegt ganz besonders bevorzugt im gleichen Temperaturbereich plus/minus 5°C wie die Mindestfilmbildetemperatur.

Die Verdampfung des Wassers in der Sprühvorrichtung kann sowohl bei Normaldruck als auch bei 0.06 bis 0.12 M Pa erfolgen.

Bei der Durchführung der Sprühverfahren können den wässrigen Polymerdispersionen auch polymere Sprühhilfsmittel wie Polyvinylalkohole, Mischungen aus Polyvinylalkohol und einem aus Polyethylenglykol als Pfropfgrundlage und Polyvinylalkohol-Seitenketten bestehenden Pfropfcopolymer (kommerziell verfügbar als Kollicoat® Protect), Polyvinylpyrrolidone,alkylierte und/oder hydroxyalkylierte Cellulosen, Stärkederivate, Ligninsulfonate, Polyacrylsäuren oder Polyacrylamide zugegeben werden. Geeignete Mengen solcher Sprühhilfsmittel liegen im Bereich von 0,1 bis 30, vorzugsweise 1 bis 10 Gew.-%, bezogen auf den Feststoffgehalt.

Weiterhin können den wässrigen Polymerdisperionen auch Antiblockiermittel zugegeben werden. Geeignete Antiblockiermittel sind z.B. Aluminium-Silikate wie Bentonit, weiterhin Kieselgur, kolloidale Kieselsäure, gefällte Kieselsäure, Diaatomeenerde, Calciumcarbonat, Titandioxid, Zinkoxid, Magnesiumsilikate wie Talkum oder Tricalciumphosphat. Geeignete Mengen solcher Antiblockiermittel liegen im Bereich von 0,1 bis 15, vorzugsweise 0,5 bis 5 Gew.-%, bezogen auf den Feststoffgehalt.

Prinzipiell können den wässrigen Polymerdispersionen auch übliche Coatinghilfsstoffe hinzugefügt werden. Geeignete Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Antioxidantien, Porenbildner, Neutralisierungsmittel, Glanzmittel, Farbstoffe, Pigmente, Desinfektions- oder Konservierungsmittel, Verdickungsmittel oder Weichmacher. Geeignete Hilfsmittel sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Wie bereits erwähnt betrifft eine Ausführungsform der Erfindung die konventionelle Sprühtrocknung, bei der die zu trocknende wässrige Polymerdispersion zerstäubt und im Gasstrom des Trocknungsgases getrocknet und auf diese Weise in Pulverform überführt wird.

Gemäß einer weiteren Ausführungsform kann die Überführung in Pulver durch eine Sprühgranulierung erfolgen. Dazu wird die zu trocknende wässrige Polymerdispersion ebenfalls zerstäubt und die erzeugten Tröpfchen gelangen dann in einem Wirbelbett in Kontakt mit vorgelegten Saatpartikeln, Durch dieses Inkontaktbringen der Saatpartikel mit den Tröpfchen der wässrigen Polymerdispersion wachsen die Saatteilchen zu größeren Granulatteilchen heran, wobei sich eine zwiebelschalenartige Struktur um das als Saatgut verwendete Teilchen ausbildet.

Gemäß einer bevorzugten Ausführungsform der Erfindung erfolgt die Überführung in die Pulverform mit Hilfe der agglomerierenden Sprühtrocknung. Dabei wird die Polymerdispersion in einem Sprühturm wie oben beschrieben zerstäubt, wobei gleichzeitig Feinstaub, der aus der Trocknungszone abgeführt wird, in die Zerstäubungszone, in der die wässrige Polymerdispersion in Form von feinen Tröpfchen vorliegt, eingeblasen wird. Die Feinstaubpartikel werden dabei zu größeren Aggregaten mit einer brombeerförmigen Struktur verklebt. Zusätzlich kann auch noch ein Wirbelbett angeschlossen werden, in dem der Wassergehalt der gebildeten Teilchen weiter reduziert werden kann. Die resultierenden Aggregate können Teilchengrößen von 150 bis 1000 µm bevorzugt von 200 bis 500 µm aufweisen. Auch bei dieser Ausführungssform wird die Eingangstemperatur mindestens 20°C und bevorzugt mindestens 40°C über der Glasübergangstemperatur, und gemäß einer Ausführungsform auch mindestens 20°C, bevorzugt mindestens 40 °C, über der dynamischen Einfriertemperatur und mindestens 20°C bevorzugt mindestens 40 °C über der Mindestfilmbildetemperatur des Polymers und die Ausgangstemperatur des Trocknungsgases aus der Sprühvorrichtung bei 40 bis 85°C vorzugsweise bei 45 bis 70°C des Polymers gewählt. Bevorzugt wird die Eingangstemperatur des Trocknungsgases in die Sprühvorrichtung bei 100 bis 140°C und die Ausgangstemperatur des Trocknungsgases aus der Sprühvorrichtung bei 45 bis 70°C gehalten. Besonders bevorzugt wird die Eingangstemperatur des Trocknungsgases in die Sprühvorrichtung bei 110 bis 130°C und die Ausgangstemperatur des Trocknungsgases aus der Sprühvorrichtung bei 50 bis 60°C gehalten. Die durch Sprühagglomeration erhaltenen brombeerförmigen Strukturen sind nahezu staubfrei und zeigen ein besonders vorteilhaftes Verhalten bei der Redispergierung.

Bei allen vorstehend genannten Ausführungsformen können während des Sprühprozesses Sprühhilfsmittel wie z.B. Aluminium-Silikate wie Bentonit, Kieselgur, kolloidale Kieselsäure, gefällte Kieselsäure, Diaatomeenerde, Calciumcarbonat, Titandioxid, Zinkoxid, Magnesiumsilikate wie Talkum oder Tricalciumphosphat in Mengen von 0,1 bis 15, vorzugsweise 0,5 bis 5 Gew.-%, bezogen auf das Polymerpulver in den Sprühtum eingeblasen werden.

Der Restlösemittelgehalt beträgt üblicherweise nicht mehr als 5 Gew.-%, bezogen auf den Feststoffgehalt des Pulvers.

Insgesamt richten sich die Teilchengrößen des durch Sprühverfahren entstehenden Pulvers nach der jeweiligen Ausführungsform. Bei einer normalen Sprühtrocknung können mittlereTeilchengrößen von 10 bis 150 µm erzielt werden. Bei einer Sprühgranulation wie beispielsweise einer Sprühwirbelschichttrocknung können größere Teilchengrößen von 150 bis zu 1000 µm erreicht werden. Bei der agglomerierenden Sprühtrocknung können Teilchengrößen von 150 bis 1000 µm erzielt werden.

Gemäß einer weiteren Ausführungsform werden dem Polymer Säuren zugesetzt. Vorzugsweise werden solche Mengen an Säure zugesetzt, dass die basischen Gruppen teilweise in Form der Säuresalze vorliegen. Bevorzugt werden 1 bis 20 mol-%, besonders bevorzugt 2 bis 15 mol-% der basischen Gruppen neutralisiert. Dies kann vor oder nach der Sprühtrocknung erfolgen. So kann beispielsweise die Säure vor der Sprühtrocknung zu der wässrigen Polymerdispersion gegeben werden. Gemäß einer anderen Ausführungsform kann die Säure auch vor oder während der Redispergierung zugesetzt werden. Erfolgt die Einarbeitung der Säure vor der Sprühtrocknung, so kann diese mit üblichen Verfahren in die wässrige Dispersion eingerührt werden. Bei Zugabe nach der Sprühtrocknung erfolgt die Einarbeitung der Säure in das Polymerpulver so, dass zunächst das Polymerpulver in Wasser mittels eines einfachen Rührers grob vordispergiert wird, anschließend die Säure zugesetzt wird und durch Weiterrühren die vollständige Redispergierung erreicht wird. Die Redispergierung ist sehr schnell und so liegen schon nach 10min feinteilige Dispergate vor. In einer modifizierten Vorgehensweise kann auch zunächst die Säure in Wasser vorgelegt werden und hierzu unter Rühren das Polymerpulver gegeben werden. Ferner ist es möglich, zunächst Polymerpulver und Säure zu mischen und diese Pulvermischung in Wasser einzutragen.
Als Säuren eignen sich anorganische Säuren oder saure Salze wie Kohlensäure (Einpressen von Kohlendioxid), Ammoniumhydrogencarbonat, Natriumhydrogencarbonat, Salzsäure, Schwefelsäure oder Phosphorsäure oder Phosphorsäuresalze wie Natriumdihydrogenphosphat. Weiterhin eignen sich organische Säuren wie Weinsäure, Citronensäure, Milchsäure, Glykolsäure, Äpfelsäure, Malonsäure, Maleinsäure, Bernsteinsäure, Fumarsäure, Asparaginsäure, Glutaminsäure, Gluconsäure oder weitere physiologisch verträgliche Säuren. Selbstverständlich kommen auch polymere Säuren auf natürlicher und/oder synthetischer Basis in Frage. Die genannten Säuren eigenen sich für alle beschriebenen Ausführungsformen.

Gemäß einer weiteren Ausführungsform der Erfindung werden Säuren, die unter den Bedingungen des Sprühverfahrens zersetzt werden oder verdampfen, eingesetzt. Nach dieser Ausführungsform liegen die Polymere vor und während des Sprühverfahrens in neutralisierter oder teilneutralisierter Form vor, während im entstehenden Pulver wieder die freie basische Form vorliegt.
Welche Gewichtsmengen an Surren im Einzelnen verwendet werden, richtet sich nach dem jeweiligen Molekulargewicht und dem oben beschriebenen angestrebten Neutralisationsgrad.

Bevorzugt wird die Behandlung mit Säuren so durchgeführt, dass der pH-Wert der wässrigen Dispersion, des Pulvers oder des in Wasser redispergierten Pulvers im Bereich von 5 bis 9 liegt.

Besonders bevorzugt erfolg die Zugabe der Säure oder des sauren Salzes so, dass der pH-Wert der wässrigen Dispersion, des Pulvers oder des in Wasser redispergierten Pulvers im Bereich von 6 bis 8 liegt.

Die Herstellung von Überzugsmitteln kann z. B. durch inniges Vermischen einer durch Redispergieren des erfindungsgemäß erhaltenen Polymerpulvers zu einer wässrigen Polymerdispersion, der vorzugsweise mindestens ein weiterer Hilfsstoff zugesetzt wird, erfolgen.

Gemäß einer bevorzugten Ausführungsform wird während oder nach dem Sprühverfahren dem entstehenden Polymerpulver Siliciumdioxid zugesetzt.

Geeignete zusätzliche Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Antiklebemittel, Stabilisatoren, Antioxidantien, Porenbildner, Neutralisierungsmittel, Glanzmittel, Farbstoffe, Pigmente, Desinfektions- oder Konservierungsmittel, Verdickungsmittel, Weichmacher etc. Solche Stoffe sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Gemäß einer Ausführungsform der Erfindung wird zur Herstellung des Überzugsmittels das N,N-Diethylaminoethylmethacrylat-basierende Polymerpulver vor der Redisergierung in Wasser gemahlen. Die Mahlung kann auch in Gegenwart der genannten zusätzlichen Hilfsstoffe erfolgen.

Übliche Mengen der Hilfsstoffe liegen in einem Bereich von jeweils 0 bis 70 Gew.-%, bevorzugt 0 bis 60 Gew.-%, insbesondere 1 bis 50 Gew.-%, bezogen auf das Gesamtgewicht des Feststoffes des Überzugsmittels.

Das aus den erfindungsgemäßen Pulvern erhaltene Überzugsmittel kann aber auch in Pulverform auf die pharmazeutischen Dosierungsformen aufgetragen werden.
Der Auftrag kann auch in wässriger Form durch Granulieren, Gießen, Ausstreichen oder mittels Sprühauftrag erfolgen.

Bevorzugt ist die Anwendung als durch Redispergierung erhaltene wässrige Polymerdispersion. Grundsätzlich eignet sich jede Dispergiervorrichtung für die Redispergierung. Dabei erfolgt die Redispergierung vorzugsweise unter Anwendung niedriger Scherkräfte, vorzugsweise mittels eines Blatt, Propeller, Ankerrührers oder eines vergleichbaren Rührwerkzeuges. Die erfindungsgemäßen Polymerpulver redispergieren hierbei spontan und schnell. Die Redispergierung der Polymerpulver in Wasser ist üblicherweise in 10 min abgeschlossen.

Diesen redispergierten Zubereitungen können für den Coatingauftrag erforderliche weitere Komponenten zugesetzt werden. Solche Komponenten sind insbesondere Weichmacher wie z.B. Triethylcitrat, Tributylcitrat, Diethylsebacat, Dibutylsebacat, Acetyltriethylcitrat.

Überraschenderweise überstehen die feinteiligen Dispersionen auch sehr hohe Scherkräfte wie beispielsweise in einer Rotor-Stator-Vorrichtung, die auch Ultra-turrax genannt wird oder einer Kolloidmühle. Die Eintragung hoher Scherkräfte wird in einer Rotor-Stator-Vorrichtung über die Umdrehungszahl der Vorrichtung geregelt. Vorzugsweise erfolgt die Redispergierung mit Hilfe einer Dispergiervorrichtung bei < 5000 Upm. Dieses Verfahren ist insbesondere von Vorteil wenn zusätzlich in die Dispersion weitere grobteilige Zusatzstoffe oder agglomerierte Zusatzstoffe eingearbeitet werden müssen, die einer besonderen Zerkleinerung bedürfen. Es entfällt somit die separate Zerkleinerung dieser Zusatzstoffe in Wasser und spätere Zugabe zum redispergierten Polymerpulver.
In einer besonderen Ausführungsform werden aus den erfindungsgemäßen redispergierbaren Polymerpulvern durch Vermischen mit weiteren üblichen vorstehend beschriebenen Coatingbestandteilen bzw. Zusatzstoffen sogenannte ready-to use Zubereitungen hergestellt, die alle erforderlichen Bestandteile eine Coatings enthalten. Diese liegen in Pulver- oder Granulatform vor. Der Anwender braucht diese zur Herstellung einer sprühfertigen Suspension nur noch in Wasser einzurühren. Die Herstellung dieser ready-to-use Zubereitungen erfolgt durch trockenes Mischen, Vermahlen, Kompaktieren oder Granulieren der Bestandteile mit einer Granulierflüssigkeit gefolgt von einem Abtrocknungsschritt. Insbesondere können auf diese Weise Säuren oder saure Salze, die die Redispergierung unterstützen, eingearbeitet werden.

Wenn nicht anders angegeben, handelt es sich bei allen Angaben im Rahmen der vorliegenden Erfindung zur mittleren Teilchengröße von Pulvern im Mikrometerbereich um das mittels Lichtbeugung bestimmte Volumenmittel der Teilchendurchmesser (d4,3-Wert).

Die erfindungsgemäßen Überzugsmittel können zusätzlich wenigstens eine weitere Polymerkomponente enthalten. Dabei können Mischungen aus wenigstens zwei Dispersionen, wenigstens einer Dispersion und wenigstens einer Lösung, wenigstens einer Dispersion und wenigstens einem Pulver, wenigstens zwei Pulvern, etc. zum Einsatz kommen.

Die erfindungsgemäßen Überzugsmittel eignen sich für Dosierungsformen grundsätzlich beliebiger pharmazeutischer Wirkstoffe, die vorzugsweise in isolierter oder geschützter Form verabreicht werden können, wie Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen- Darmtherapeutika, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika, Nutraceuticals, Vitamine, Carotinoide und Aminosäuren.

Beispiele geeigneter Wirkstoffe sind: Acarbose, Nichtsteroidale Antirheumatika, Herzglykoside, Acetylsalicylsäure, Virustatika, Aclarubicin, Aciclovir, Cisplatin, Actinomycin, α-und β-Sympatomimetika, Allopurinol, Alosetron, Alprostadil, Prostaglandine, Amantädin, Ambroxol, Amlodipin, Methotrexat, 5-Aminosalicylsäure, Amitriptylin, Amoxicillin, Anastrozol, Atenolol, A-torvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und. Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridamol, Domperidon und Domperidonderivate, Donepzil, Dopamin, Doxazosin, Doxorubicin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerofderivate, Hypothalamushormone, Goserelin, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat, Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate ; Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralin, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Antiöstrogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zopiclon, Zotepin und dergleichen.

Die Wirkstoffe können gewünschtenfalls auch in Form ihrer pharmazeutisch akzeptablen Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemäßen Zusammensetzungen auch zwei oder mehrere pharmazeutische Wirkstoffe enthalten.

Erfindungsgemäß können die Überzugsmittel zur Beschichtung von Extrudaten, Minitabletten, Kapseln, Weichkapseln, Granulaten, Pellets, Mikropellets, Mikrokapseln, Nanokapseln oder Kristallen eingesetzt werden.

Zur Herstellung von Dosierungsformen können die überzogenen Granulate, Pellets, Mikropellets, Mikrokapseln, Kristalle mit geeigneten Hilfsstoffen abgemischt und zu Tabletten verpresst werden, die im wässrigen Milieu der Mundhöhle zerfallen und die gecoateten feinen Formlinge wieder freigeben. Besondere Bedeutung haben hierbei so genannte oral dispersibles, d. h. Tabletten, die im Mund innerhalb von kurzer Zeit zerfallen und die geschmacksmaskierten kleinen Formlinge freisetzen.

Weiterhin können die Überzugsmittel auch vorteilhaft zum Überziehen von Tabletten eingesetzt werden.

Wirkstoffklassen und Substanzen, die oftmals einen unangenehmen bitteren Geschmack hervorrufen können und sich vorteilhaft erfindungsgemäß formulieren lassen, sind z. B.:
Analgetika und Antirheumatika, wie Paracetamol, Diclofenac, Aceclofenac, Ibuprofen, Ketoprofen, Flubiprofen, Acetylsalicylsäure, Levacetylmethadol und Oxycodon;
Psychopharmaka, wie Promethazine, Donepezil, Modafinil, Nefazodon, Reboxetin, Sertindol und Sertralin;
Antibiotika, wie Erythromycin, Roxithromycin, Clarithromycin, Grepafloxacin, Ciprofloxacin, Levofloxacin, Sparfloxacin, Trovafloxacin und Nevirapin;
Betablocker, wie Propranolol, Metoprolol, Bisoprolol und Nebivolol;
Antidiabetika, wie Metformin, Miglitol und Repaglinid;
H₁-Antihistaminika, wie Diphenhydramin, Fexofenadin und Mizolastin;
H₂ Antihistaminika, wie Cimetidin, Famotidin, Roxatidin, Nizatidin, Ticlopidin, Cetirizin und Ranitidin;
Vitamine wie Thiaminnitrat sowie Chinidin-Sulfat, Amyloprilose-HCl, Pseudoephedrin-HCl, Sildenafil, Topiramat, Granisetron, Rebamipide, Chinin- HCl, etc.
Auch verschiedene Salze dieser Wirkstoffe können entsprechend formuliert werden.

Die hervorragende Geschmacksmaskierung resultiert aus der Unlöslichkeit der erfindungsgemäßen Polymeren bei pH-Werten größer 6 und der schnellen Löslichkeit bei pH-Werten unter 6. Das heißt im Speichel (pH-Wert: 7,2) sind entsprechend überzogene Formen sehr lange stabil und es erfolgt kein Kontakt des bitteren Arzneistoffs mit der Mundschleimhaut, aber im Magen bei pH-Werten von 1 bis 5 erfolgt eine sehr schnelle Freisetzung des Wirkstoffs. Die Auflösung ist dabei so schnell, dass kein Unterschied im Wirkungseintritt vorhanden ist, verglichen mit einer nicht gecoateten Form. In der Regel lösen sich Filmüberzüge eines erfindungsgemäßen Polymers innerhalb von 5 min in Magensaft auf, wohingegen sie in Phosphatpuffer pH 7,2 über 2 Stunden stabil sind. Überraschenderweise lösen sich die Filmüberzüge auch in Medien mit pH-Werten von 4,5 relativ schnell, so dass die daraus hergestellten Darreichungsformen auch bei anaciden Patienten bzw. Patienten, die mit Antacida behandelt werden, eine schnelle Wirkung entfalten. Diese hervorragenden Anwendungseigenschaften der Überzugsmittel bleiben auch nach der Überführung in Pulver und Redispergierung der Pulver erhalten.

Überraschenderweise gelingt mit Hilfe des erfindungsgemäßen Verfahrens die Überführung der wässrigen Polymerdispersion in freifliessende Pulver, ohne das es zu größeren Agglomerationen und Ablagerungen in der Sprühvorrichtung kommt.

Überraschend war für den Fachmann auch, dass sich die agglomerierende Sprühtrocknung so vorteilhaft anwenden liess. Die Anwendbarkeit dieser Technologie ist umso überraschender, als hierbei feine Pulverteilchen wieder vor die Sprühdüse geblasen werden, somit Kontakt mit heißer Zuluft erhalten, und trotzdem redispergierbar bleiben.

Für den Fachmann war dies im Hinblick auf die Empfehlungen des Standes der Technik bezüglich Trocknungsmethode oder Temperaturführung der Trocknungsgase nicht zu erwarten gewesen. Überraschend war auch, dass die redispergierten feinteiligen Dispersionen den Einsatz hoher Scherkräfte überstehen, da der Fachmann bei hohen Scherkräften normalerweise ein Koagulieren der Dispersion erwartet hätte. Feinteilige Dispersionen sind nämlichsonst hinlänglich dafür bekannt, sehr empfindlich gegenüber starken Scherbeanspruchungen zu sein.

Das erfindungsgemäße Verfahren führt demgemäß zu Polymerpulvern mit einer guten Teilchengrößenverteilung und guten anwendungstechnischen Eigenschaften wie beispielsweise der Fliessfähigkeit. Bei der Verwendung zur Herstellung von Überzugsmitteln lassen sich die Pulver sehr vorteilhaft zu feinteiligen Dispersionen redispergieren. Häufig bestehen zwischen den Teilchengrößen des Redispergates und der originären Dispersion nur geringe Unterschiede.

### Beispiele

Verwendete Abkürzungen:
Glasübergangstemperatur: Tg

Alle Angaben in % beziehen sich auf Gew.-%.

Die Herstellung des Polymeren erfolgt analog Beispiel 1 der WO 2009/016258.
Polymer A: Methylmethacrylat/Diethylaminoethylmethacrylat, Gew.v. 60:40, K-Wert 50, Tg 62°C
Polymer B: Methylmethacrylat/Diethylaminoethylmethacrylat, Gew.v. 55:45, K-Wert 49, Tg 57°C
Polymer C: Methylmethacrylat/Diethylaminoethylmethacrylat, Gew.v. 53:47, K-Wert 52, Tg 55°C

Die K-Werte wurden 0.1 gew.-%ig in NMP gemessen. Die Polymere wurden als 30 gew.-%ige wässrige Dispersionen mit einem pH-Wert von 9+/- 0.3 eingesetzt. Die mittleren Partikelgrößen der Primärdispersionen betrugen 128, 127 und 131 nm. Die Glasübergangstemperaturen wurden mittels DSC bei einer Heizrate von 20 °K/min bestimmt. Die Mindestfilmbildetemperatur entsprach im Rahmen der Messgenauigkeit von +/- 5°C der Tg.

Bei der Bestimmung der mittleren Teilchengrößen der Pulver wurde der (d4,3)-Wert durch Lichtbeugung mittels eines Malvern Mastersizers 2000 bestimmt.

Bei der Bestimmung der mittleren Teilchengrößen der redispergierten Pulver mittels Lichtstreuung wurde der Wert mittels eines "Malvern Zetasizer nano-s" als Intensitätsmittel bestimmt.

### Beispiel 1

1000 ml einer wässrigen Dispersion des Polymers B mit einem Feststoffgehalt von 30% wurden unter Rühren mit 72,9 ml 1 molarer Salzsäure vermischt. Dies entspricht einem Neutralisationsgrad von 10 mol-%. Diese teilneutralisierte Dispersion wurde in einem FSD-Sprühturm sprühgetrocknet, wobei die Zerstäubung über eine 1,2 mm Zweistoffdüse bei 2,5 bar Zerstäubungsdruck erfolgte. Die Zulufttemperatur betrug 109°C und die Ablufttemperatur 54°C. Der Feinanteil wurde während der Sprühtrocknung abgetrennt und wieder vor die Sprühdüse geblasen, so dass sprühgetrocknete Teilchen mit einer mittleren Teilchengröße von 180 µm resultierten. Das sprühgetrocknete Produkt wurde in Wasser zu einer Sprühsuspension mit 20% Feststoffgehalt durch Rühren mit einem Blattrührer für 15 min redispergiert. Die Messung der Teilchengröße mittels Lichtstreuungergab einen Wert von 128 nm.

### Beispiel 2

1000 ml einer wässrigen Dispersion des Polymers A mit einem Feststoffgehalt von 30% wurden in einem-Sprühturm sprühgetrocknet. Die Zerstäubung erfolgte hierbei über eine 1,2 mm Zweistoffdüse bei 3,0 bar Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 108°C und die Ablufttemperatur 57°C. Die mittlere Teilchengröße der Pulver betrug 30 µm.
100g sprühgetrocknetes Produkt wurden in 900 ml Wasser eingetragen, in dem vorher 2,55g Bernsteinsäure gelöst wurden. Die Zubereitung wurde 20 min mit einem Propellerrührer gerührt. Die Messung der Teilchengröße mittels Lichtstreuung ergab einen Wert von 135 nm.

### Beispiel 3

1000 ml einer wässrigen Dispersion des Polymers A mit einem Feststoffgehalt von 30% wurden in einem-Sprühturm sprühgetrocknet. Die Zerstäubung erfolgte hierbei über eine 1,2 mm Zweistoffdüse bei 3,0 bar Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 110°C und die Ablufttemperatur 59°C. Die mittlere Teilchengröße der Pulver betrug 32 µm.

150g sprühgetrocknetes Produkt wurden in 850 ml Wasser eingetragen und die Zubereitung 20 min mit einem Ultra-turrax bei 12.000 Upm behandelt. Die Messung der Teilchengröße mittels Lichtstreuung ergab einen Wert von 270 nm.

### Beispiel 4

1000 ml einer wässrigen Dispersion des Polymers B mit einem Feststoffgehalt von 30% wurden unter Rühren mit 36,5 ml 1 molarer Salzsäure vermischt. Dies entspricht einem Neutralisationsgrad von 5 mol-%. Diese teilneutralisierte Dispersion wurde in einem FSD-Sprühturm sprühgetrocknet, wobei die Zerstäubung über eine 1,2 mm Zweistoffdüse bei 3,0 bar Zerstäubungsdruck erfolgte. Die Zulufttemperatur betrug 130°C und die Ablufttemperatur 59°C. Der Feinanteil wurde während der Sprühtrocknung abgetrennt und wieder vor die Sprühdüse geblasen, so dass sprühgetrocknete Teilchen mit einer mittleren Teilchengröße von 200 µm resultierten. Während des Sprühprozesses wurde kolloidales Siliciumdioxid mit einer BET-Oberfläche von 200 m2/g in einer Menge von 0,5% bezogen auf die Gesamtmasse des Polymerpulvers in den Turm eingeblasen.

Das sprühgetrocknete Produkt wurde in Wasser zu einer Sprühsuspension mit 20% Feststoffgehalt durch Rühren mit einem Blattrührer für 20 min redispergiert. Die Messung der Teilchengröße mittels Lichtstreuung ergab einen Wert von 130 nm.

### Beispiel 5

1000 ml einer wässrigen Dispersion des Polymers C mit einem Feststoffgehalt von 30% wurden unter Rühren mit 1,31 g Malonsäure vermischt. Dies entspricht einem Neutralisationsgrad von 5 mol-%. Diese teilneutralisierte Dispersion wurde mit 200 ml einer 40%igen Suspension von Talkum vermischt und in einem FSD-Sprühturm sprühgetrocknet, wobei die Zerstäubung über eine 1,2 mm Zweistoffdüse bei 3,0 bar Zerstäubungsdruck erfolgte. Die Zulufttemperatur betrug 135°C und die Ablufttemperatur 61 °C. Der Feinanteil wurde während der Sprühtrocknung abgetrennt und wieder vor die Sprühdüse geblasen, so dass sprühgetrocknete Teilchen mit einer mittleren Teilchengröße von 210 µm resultierten.

Das sprühgetrocknete Produkt wurde in Wasser zu einer Sprühsuspension mit 20% Feststoffgehalt durch Rühren mit einem Blattrührer für 15 min redispergiert. Die Messung der Teilchengröße mittels Lichtstreuung ergab für die Polymerteilchen einen Wert von 141 nm.

### Beispiel 6

1000 ml einer wässrigen Dispersion des Polymers B mit einem Feststoffgehalt von 30% wurden unter Rühren mit 200 g Wasser, 100 g Talkum, 20 g rotes Eisenoxid und 10g Polyvinylalkohol und 36,5 ml 1 molarer Salzsäure versetzt. Diese Zubereitung wurde mittels eines Ultra-turrax 20 min bei 12.000 Upm homogenisiert und anschließend in einem FSD-Sprühturm sprühgetrocknet, wobei die Zerstäubung über eine 1,2 mm Zweistoffdüse bei 3,0 bar Zerstäubungsdruck erfolgte. Die Zulufttemperatur betrug 138°C und die Ablufttemperatur 62°C. Der Feinanteil wurde während der Sprühtrocknung abgetrennt und wieder vor die Sprühdüse geblasen, so dass sprühgetrocknete Teilchen mit einer mittleren Teilchengröße von 220 µm resultierten.

Das sprühgetrocknete Produkt wurde in Wasser zu einer Sprühsuspension mit 20% Feststoffgehalt durch Rühren mit einem Blattrührer für 30 min redispergiert. Die Messung der Teilchengröße mittels Lichtstreuung ergab für die Polymerteilchen einen Wert von 133 nm.

### Beispiel 7

1000 ml einer wässrigen Dispersion des Polymers B mit einem Feststoffgehalt von 30% wurden unter Rühren mit 15g Polyvinylalkohol, 6g Dokusat-Natrium und 3 g kolloidalem Siliciumdioxid versetzt. Diese Dispersion wurde in einem FSD-Sprühturm sprühgetrocknet, wobei die Zerstäubung über eine 1,2 mm Zweistoffdüse bei 3,0 bar Zerstäubungsdruck erfolgte. Die Zulufttemperatur betrug 105°C und die Ablufttemperatur 48°C. Der Feinanteil wurde während der Sprühtrocknung abgetrennt und wieder vor die Sprühdüse geblasen. Während des Sprühprozesses wurde kolloidales Siliciumdioxid mit einer BET-Oberfläche von 200 m2/g in einer Menge von 1,0% bezogen auf die Gesamtmasse des Polymerpulvers in den Turm eingeblasen. In einem direkt an den Sprühturm angeschlossenem Wirbelbett wurde das Pulver bei 45°C nachgetrocknet. Die sprühgetrockneten Teilchen wiesen eine mittlere Teilchengröße von 210 µm auf.

Das sprühgetrocknete Produkt wurde in Wasser zu einer Sprühsuspension mit 15% Feststoffgehalt durch Rühren mit einem Blattrührer für 20 min redispergiert. Die Messung der Teilchengröße mittels Lichtstreuung ergab einen Wert von 170 nm.

### Beispiel 8

In 1000 ml einer wässrigen Dispersion des Polymers A mit einem Feststoffgehalt von 30% wurden 3,5 g Natriumdihydrogenphosphat gelöst und anschließend in einem-Sprühturm sprühgetrocknet. Die Zerstäubung erfolgte hierbei über eine 1,2 mm Zweistoffdüse bei 3,0 bar Zerstäubungsdruck. Das Trocknungsgas wurde tangential im Eingangsbereich des Sprühtrockners zugeführt und das getrocknete Produkt in einem Zyklon abgeschieden. Die Zulufttemperatur betrug 115°C und die Ablufttemperatur 55°C. Die mittlere Teilchengröße betrug 35 µm.

Nach der Redispergierung dieser Zubereitung in Wasser zu einer 15%igen Suspension mittels eines Propellerrührers über 15 min ergab sich eine Teilchengröße der Polymerteilchen von 138 nm.

### Beispiel 9

100 g Polymerpulver hergestellt nach Beispiel 3 wurden mit 50 g feinst gemahlenem Talkum, 4g Indigotinlack und 2g Bernsteinsäure in einem Turbulamischer vermischt.

Nach der Redispergierung dieser Zubereitung in Wasser zu einer 15%igen Suspension mittels eines Propellerrührers ergab sich eine Teilchengröße der Polymerteilchen von 150 nm.

### Beispiel 10

Die nach Beispiel 9 hergestellte Zubereitung wurde mit 15 g Triethylcitrat versetzt, zwei Stunden gerührt und durch Versprühen auf Tablettenkerne aufgebracht.

**Sprühbedingungen:**

| Maschine | Horizontaltrommelcoater |
|---|---|
| Zulufttemperatur | 54°C |
| Sprühdruck | 0.2 MPa |
| Formierluftdruck | 0.1 MPa |
| Sprühdüse | Schlick 930 / 1mm |
| Zuluftmenge | 200 m³/h |
| Sprührate | 30 g/min. |

## Patentansprüche

1. Verfahren zur Herstellung von pulverförmigen Überzugsmitteln aus wässrigen Polymerdispersionen, enthaltend
i) als Komponente A ein durch radikalische Polymerisation erhaltenes Polymer aus
a) N,N-Diethylaminoethylmethacrylat, und
b) wenigstens eine radikalisch polymerisierbare Verbindung, ausgewählt unter Estern α,β-ethylenisch ungesättigter Mono- und Dicarbonsäuren mit C₁-C₈-Alkanolen,
**dadurch gekennzeichnet, dass** die wässrige Polymerdispersion durch Sprühverfahren in Gegenwart eines Trocknungsgases in Pulver überführt wird, wobei die Eingangstemperatur des Trocknungsgases in die Sprühvorrichtung mindestens 20°C über der Glasübergangstemperatur, bestimmt nach der Methode wie beschrieben auf Seite 5, Zeilen 33-36, liegt und mindestens 20°C über der Mindestfilmbildetemperatur, bestimmt nach der Methode wie beschrieben in der Beschreibung des Polymeren liegt und die Ausgangstemperatur des Trocknungsgases aus der Sprühvorrichtung bei 40 bis 85°C gehalten wird.

2. Verfahren nach Anspruch 1, **dadurch gekennzeichnet, dass** die Eingangstemperatur des Trocknungsgases in die Sprühvorrichtung mindestens 40°C über der Glasübergangstemperatur liegt und mindestens 40°C über der Mindestfilmbildetemperatur des Polymeren liegt.

3. Verfahren nach einem der Ansprüche 1 bis 2, **dadurch gekennzeichnet, dass** die Ausgangstemperatur des Trocknungsgases aus der Sprühvorrichtung bei 45 bis 70 °C gehalten wird.

4. Verfahren nach einem der Ansprüche 1 bis 3, **dadurch gekennzeichnet, dass** die Eingangstemperatur des Trocknungsgases in die Sprühvorrichtung bei 100 bis 140 °C und die Ausgangstemperatur des Trocknungsgases aus der Sprühvorrichtung bei 45 bis 70°C gehalten wird.

5. Verfahren nach einem der Ansprüche 1 bis 4, **dadurch gekennzeichnet, dass** die Eingangstemperatur des Trocknungsgases in die Sprühvorrichtung bei 110 bis 130°C und die Ausgangstemperatur des Trocknungsgases aus der Sprühvorrichtung bei 50 bis 60°C gehalten wird.

6. Verfahren nach einem der Ansprüche 1 bis 5, **dadurch gekennzeichnet, dass** die Ausgangstemperatur des Trocknungsgases aus der Sprühvorrichtung im Bereich von plus/minus 5°C der Mindestfilmbildetemperatur gehalten wird.

7. Verfahren nach einem der Ansprüche 1 bis 6, **dadurch gekennzeichnet, dass** das Sprühverfahren als Sprühtrocknung durchgeführt wird.

8. Verfahren nach einem der Ansprüche 1 bis 7, **dadurch gekennzeichnet, dass** das Sprühverfahren als agglomerierende Sprühtrocknung durchgeführt wird.

9. Verfahren nach einem der Ansprüche 1 bis8, **dadurch gekennzeichnet, dass** der wässrigen Polymerdispersion vor dem Sprühverfahren eine Säure oder ein anorganisches saures Salz zugesetzt wird.

10. Verfahren nach einem der Ansprüche 1 bis9, **dadurch gekennzeichnet, dass** nach dem Sprühverfahren dem entstandenen Pulver eine Säure oder ein anorganisches saures Salz zugesetzt wird.

11. Verfahren nach einem der Ansprüche 1 bis10, **dadurch gekennzeichnet, dass** nach dem Sprühverfahren das entstandenen Pulver in Wasser redispergiert und mit einer Säure oder einem anorganischen sauren Salz versetzt wird.

12. Verfahren nach einem der Ansprüche 1 bis 11, **dadurch gekennzeichnet, dass** als Säure eine anorganische Säure oder ein saures Salz davon zugesetzt wird.

13. Verfahren nach einem der Ansprüche 1 bis12, **dadurch gekennzeichnet, dass** als Säure eine organische Säure zugesetzt wird.

14. Verfahren nach einem der Ansprüche 1 bis13, **dadurch gekennzeichnet, dass** als Säure eine Säure oder ein anorganisches saures Salz davon zugesetzt wird, die unter den Bedingungen des Sprühverfahrens zersetzt oder verdampft wird.

15. Verfahren nach einem der Ansprüche 1 bis14, **dadurch gekennzeichnet, dass** durch Zugabe der Säure oder des anorganischen sauren Salzes der pH-Wert der wässrigen Dispersion, des Pulvers oder des in Wasser redispergierten Pulvers im Bereich von 5 bis 9 liegt.

16. Verfahren nach einem der Ansprüche 1 bis15, **dadurch gekennzeichnet, dass** durch Zugabe der Säure oder des anorganischen sauren Salzes der pH-Wert der wässrigen Dispersion, des Pulvers oder des in Wasser redispergierten Pulvers im Bereich von 6 bis 8 liegt.

17. Verfahren nach einem der Ansprüche 1 bis 16, **dadurch gekennzeichnet, dass** der wässrigen Polymerdispersion vor dem Sprühverfahren ein weiterer Hilfsstoff zugesetzt wird.

18. Verfahren nach einem der Ansprüche 1 bis17, **dadurch gekennzeichnet, dass** während oder nach dem Sprühverfahren dem entstehenden Polymerpulver Siliciumdioxid zugesetzt wird.

19. Verwendung eines gemäß den Ansprüchen 1 bis 18 erhaltenen Polymerpulvers als Überzugsmittel für pharmazeutische Dosierungsformen.

20. Verwendung nach Anspruch19, wobei das Überzugsmittel in Form einer durch redispergieren des Polymerpulvers erhaltenen wässrigen Dispersion auf die Dosierungsform aufgebracht wird.

21. Verwendung nach Anspruch 19 oder 20, wobei die Redispergierung mit Hilfe einer Dispergiervorrichtung bei Umdrehungen von größer 5000 Upm erfolgt.

22. Verwendung nach Anspruch 19 oder 20, wobei die Redispergierung mit Hilfe einer Dispergiervorrichtung bei Umdrehungen von kleiner 1000 Upm erfolgt.

23. Verwendung nach einem der Ansprüche 19 bis 22 zur Herstellung eines Überzugsmittels in Form von Pulvern oder Granulaten, die zusätzlich Hilfsstoffe aus der Gruppe bestehend aus Aromastoffen, geschmacksverbessernden Stoffen, Süßungsmitteln, Gleitmitteln, Netzmitteln, Trennmitteln, Antiklebemitteln, Stabilisatorenn, Antioxidantien, Porenbildnern Neutralisierungsmitteln, Glanzmitteln, Farbstoffen, Pigmenten, Desinfektions- oder Konservierungsmitteln, Verdickungsmitteln und Weichmachern, enthalten.

## Claims

1. A process for producing pulverulent coating compositions from aqueous polymer dispersions, comprising
i) as component A, a polymer obtained by radical polymerization of
a) N,N-diethylaminoethyl methacrylate, and
b) at least one radically polymerizable compound selected from esters of α,β-ethylenically unsaturated mono- and dicarboxylic acids with C₁-C₈-alkanols,
wherein the aqueous polymer dispersion is converted to powder by spraying processes in the presence of a drying gas, where the entry temperature of the drying gas into the spraying apparatus is at least 20°C above the glass transition temperature, determined according to the method as described on page 5, lines 33-36, and is at least 20°C above the minimum film-forming temperature, determined according to the method as described in the description, of the polymer and the exit temperature of the drying gas from the spraying apparatus is kept at 40 to 85°C.

2. The process according to claim 1, wherein the entry temperature of the drying gas into the spraying apparatus is at least 40°C above the glass transition temperature and is at least 40°C above the minimum film-forming temperature of the polymer.

3. The process according to either of claims 1 to 2, wherein the exit temperature of the drying gas from the spraying apparatus is kept at 45 to 70°C.

4. The process according to any one of claims 1 to 3, wherein the entry temperature of the drying gas into the spraying apparatus is 100 to 140°C and the exit temperature of the drying gas from the spraying apparatus is kept at 45 to 70°C.

5. The process according to any one of claims 1 to 4, wherein the entry temperature of the drying gas into the spraying apparatus is kept at 110 to 130°C and the exit temperature of the drying gas from the spraying apparatus is kept at 50 to 60°C.

6. The process according to any one of claims 1 to 5, wherein the exit temperature of the drying gas from the spraying apparatus is kept in the range from plus/minus 5°C of the minimum film-forming temperature.

7. The process according to any one of claims 1 to 6, wherein the spraying process is carried out as spray drying.

8. The process according to any one of claims 1 to 7, wherein the spraying process is carried out as agglomerating spray drying.

9. The process according to any one of claims 1 to 8, wherein an acid or an inorganic acidic salt is added to the aqueous polymer dispersion before the spraying process.

10. The process according to any one of claims 1 to 9, wherein an acid or an inorganic acidic salt is added after the spraying process to the resulting powder.

11. The process according to any one of claims 1 to 10, wherein, after the spraying process, the resulting polymer is redispersed in water and admixed with an acid or an inorganic acidic salt.

12. The process according to any one of claims 1 to 11, wherein an inorganic acid or an acidic salt thereof is added as acid.

13. The process according to any one of claims 1 to 12, wherein an organic acid is added as acid.

14. The process according to any one of claims 1 to 13, wherein the acid added is an acid or an inorganic acidic salt thereof which is decomposed or evaporated under the conditions of the spraying process.

15. The process according to any one of claims 1 to 14, wherein, as a result of adding the acid or the inorganic acidic salt, the pH of the aqueous dispersion, of the powder or of the water-redispersed powder is in the range from 5 to 9.

16. The process according to any one of claims 1 to 15, wherein, by adding the acid or the inorganic acidic salt, the pH of the aqueous dispersion, of the powder or of the water-redispersed powder is in the range from 6 to 8.

17. The process according to any one of claims 1 to 16, wherein a further auxiliary is added to the aqueous polymer dispersion before the spraying process.

18. The process according to any one of claims 1 to 17, wherein silicon dioxide is added to the resulting polymer powder during or after the spraying process.

19. The use of a polymer powder obtained according to claims 1 to 18 as coating composition for pharmaceutical dosage forms.

20. The use according to claim 19, where the coating composition is applied to the dosage form in the form of an aqueous dispersion obtained by redispersing the polymer powder.

21. The use according to claim 19 or 20, where the redispersion takes place with the help of a dispersion apparatus at revolutions of greater than 5000 rpm.

22. The use according to claim 19 or 20, where the redispersion takes place with the help of a dispersing apparatus at revolutions of less than 1000 rpm.

23. The use according to any one of claims 19 to 22 for producing a coating composition in the form of powders or granules which additionally comprise auxiliaries from the group consisting of aroma substances, taste-improving substances, sweetening agents, glidants, wetting agents, release agents, antisticking agents, stabilizers, antioxidants, pore formers, neutralizing agents, luster agents, dyes, pigments, disinfectants or preservatives, thickeners and plasticizers.

## Revendications

1. Procédé de fabrication d'agents de revêtement en poudre à partir de dispersions aqueuses de polymères, contenant :
i) en tant que composant A, un polymère obtenu par polymérisation radicalaire de
a) du méthacrylate de N,N-diéthylaminoéthyle et
b) au moins un composé polymérisable par voie radicalaire, choisi parmi les esters d'acides mono- et dicarboxyliques α,β-éthyléniquement insaturés avec des alcanols en C₁-C₈,
**caractérisé en ce que** la dispersion aqueuse de polymère est transformée en poudre par un procédé de pulvérisation en présence d'un gaz séchant, la température d'entrée du gaz séchant dans le dispositif de pulvérisation étant au moins 20 °C au-dessus de la température de transition vitreuse, déterminée par la méthode telle que décrite à la page 5, lignes 33 à 36, et au moins 20 °C au-dessus de la température filmogène minimale, déterminée par la méthode telle que décrite dans la description, du polymère, et la température de sortie du gaz séchant du dispositif de pulvérisation étant maintenue à 40 à 85 °C.

2. Procédé selon la revendication 1, **caractérisé en ce que** la température d'entrée du gaz séchant dans le dispositif de pulvérisation est au moins 40 °C au-dessus de la température de transition vitreuse et au moins 40 °C au-dessus de la température filmogène minimale du polymère.

3. Procédé selon l'une quelconque des revendications 1 à 2, **caractérisé en ce que** la température de sortie du gaz séchant du dispositif de pulvérisation est maintenue à 45 à 70 °C.

4. Procédé selon l'une quelconque des revendications 1 à 3, **caractérisé en ce que** la température d'entrée du gaz séchant dans le dispositif de pulvérisation est de 100 à 140 °C et la température de sortie du gaz séchant du dispositif de pulvérisation est de 45 à 70 °C.

5. Procédé selon l'une quelconque des revendications 1 à 4, **caractérisé en ce que** la température d'entrée du gaz séchant dans le dispositif de pulvérisation est de 110 à 130 °C et la température de sortie du gaz séchant du dispositif de pulvérisation est de 50 à 60 °C.

6. Procédé selon l'une quelconque des revendications 1 à 5, **caractérisé en ce que** la température de sortie du gaz séchant du dispositif de pulvérisation est maintenue dans la plage de plus/moins 5 °C de la température filmogène minimale.

7. Procédé selon l'une quelconque des revendications 1 à 6, **caractérisé en ce que** le procédé de pulvérisation est réalisé sous la forme d'un séchage par pulvérisation.

8. Procédé selon l'une quelconque des revendications 1 à 7, **caractérisé en ce que** le procédé de pulvérisation est réalisé sous la forme d'un séchage par pulvérisation agglomérant.

9. Procédé selon l'une quelconque des revendications 1 à 8, **caractérisé en ce qu'**un acide ou un sel acide inorganique est ajouté à la dispersion aqueuse de polymère avant le procédé de pulvérisation.

10. Procédé selon l'une quelconque des revendications 1 à 9, **caractérisé en ce qu'**un acide ou un sel acide inorganique est ajouté à la poudre formée après le procédé de pulvérisation.

11. Procédé selon l'une quelconque des revendications 1 à 10, **caractérisé en ce que** la poudre formée après le procédé de pulvérisation est redispersée dans de l'eau et mélangée avec un acide ou un sel acide inorganique.

12. Procédé selon l'une quelconque des revendications 1 à 11, **caractérisé en ce qu'**un acide inorganique ou un sel acide de celui-ci est ajouté en tant qu'acide.

13. Procédé selon l'une quelconque des revendications 1 à 12, **caractérisé en ce qu'**un acide organique est ajouté en tant qu'acide.

14. Procédé selon l'une quelconque des revendications 1 à 13, **caractérisé en ce qu'**un acide ou un sel acide inorganique de celui-ci, qui est décomposé ou évaporé dans les conditions du procédé de pulvérisation, est ajouté en tant qu'acide.

15. Procédé selon l'une quelconque des revendications 1 à 14, **caractérisé en ce que** le pH de la dispersion aqueuse, de la poudre ou de la poudre redispersée dans de l'eau se situe dans la plage allant de 5 à 9 par l'ajout de l'acide ou du sel acide inorganique.

16. Procédé selon l'une quelconque des revendications 1 à 15, **caractérisé en ce que** le pH de la dispersion aqueuse, de la poudre ou de la poudre redispersée dans de l'eau se situe dans la plage allant de 6 à 8 par l'ajout de l'acide ou du sel acide inorganique.

17. Procédé selon l'une quelconque des revendications 1 à 16, **caractérisé en ce qu'**un adjuvant supplémentaire est ajouté à la dispersion aqueuse de polymère avant le procédé de pulvérisation.

18. Procédé selon l'une quelconque des revendications 1 à 17, **caractérisé en ce que** du dioxyde de silicium est ajouté à la poudre polymère formée pendant ou après le procédé de pulvérisation.

19. Utilisation d'une poudre polymère obtenue selon les revendications 1 à 18 en tant qu'agent de revêtement pour formes pharmaceutiques.

20. Utilisation selon la revendication 19, dans laquelle l'agent de revêtement est appliqué sur la forme pharmaceutique sous la forme d'une dispersion aqueuse obtenue par redispersion de la poudre polymère.

21. Utilisation selon la revendication 19 ou 20, dans laquelle la redispersion a lieu à l'aide d'un dispositif de dispersion à des vitesses de rotation de plus de 5 000 tours/minute.

22. Utilisation selon la revendication 19 ou 20, dans laquelle la redispersion a lieu à l'aide d'un dispositif de dispersion à des vitesses de rotation de moins de 1 000 tours/minute.

23. Utilisation selon l'une quelconque des revendications 19 à 22 pour la fabrication d'un agent de revêtement sous la forme de poudres ou de granulats, qui contiennent en outre des adjuvants du groupe constitué par les arômes, les substances améliorant le goût, les édulcorants, les lubrifiants, les agents mouillants, les agents démoulants, les agents anti-adhérents, les stabilisateurs, les antioxydants, les agents de formation de pores, les agents de neutralisation, les agents brillants, les colorants, les pigments, les désinfectants ou les conservateurs, les épaississants et les plastifiants.
